(19) Europäisches Patentamt
European Patent Office
Office européen des brevets

(11) **EP 3 117 767 B1**

(12) **EUROPEAN PATENT SPECIFICATION**

(45) Date of publication and mention
of the grant of the patent:
**07.07.2021 Bulletin 2021/27**

(51) Int Cl.:
***A61B 5/021*** (2006.01)   ***A61B 5/024*** (2006.01)
***A61B 5/08*** (2006.01)   ***A61B 5/00*** (2006.01)
***A61B 5/0205*** (2006.01)

(21) Application number: **16170035.6**

(22) Date of filing: **17.05.2016**

(54) **PROCESSING BIOLOGICAL DATA**

VERARBEITUNG BIOLOGISCHER DATEN

TRAITEMENT DE DONNÉES BIOLOGIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.07.2015 EP 15177174**

(43) Date of publication of application:
**18.01.2017 Bulletin 2017/03**

(73) Proprietor: **Preventicus GmbH
07743 Jena (DE)**

(72) Inventor: **Hübner, Thomas
07749 Jena (DE)**

(74) Representative: **Fish & Richardson P.C.
Highlight Business Towers
Mies-van-der-Rohe-Straße 8
80807 München (DE)**

(56) References cited:
**WO-A2-2009/152521     GB-A- 2 505 890**

- **GREEN GEOFFREY C ET AL: "Continuous
multiorgan variability analysis to track severity
of organ failure in critically ill patients",
JOURNAL OF CRITICAL CARE, vol. 28, no. 5, 31
October 2013 (2013-10-31), XP028711912, ISSN:
0883-9441, DOI: 10.1016/J.JCRC.2013.04.001**
- **DASH S ET AL: "Automatic Real Time Detection
of Atrial Fibrillation", ANNALS OF BIOMEDICAL
ENGINEERING, KLUWER ACADEMIC
PUBLISHERS-PLENUM PUBLISHERS, NE, vol.
37, no. 9, 17 June 2009 (2009-06-17), pages
1701-1709, XP019727015, ISSN: 1573-9686, DOI:
10.1007/S10439-009-9740-Z**
- **D BRISINDA D ET AL: "Discriminant Analysis of
Heart Rate Variability after Electrical
Cardioversion Predicts Atrial Fibrillation
Recurrence", INTERNATIONAL JOURNAL OF
CLINICAL CARDIOLOGY, 25 November 2014
(2014-11-25), XP055310974,**
- **None**

## Description

## Technical Field

The present invention relates to processing of biological data. Based on pulse waveform analysis, data pertaining to, for example, the heart rate and the respiratory rate of a human subject are determined and processed.

## Background Art

The primary causes for diseases such as heart attack and stroke are conditions that are often hard to detect and do not entail pronounced symptoms. For example, hypertension and coronary artery disease (CAD) are among the primary causes for heart attack and atrial fibrillation (AF) is one of the primary causes for stroke. Regular measurement of, for example, blood pressure, heart rate, respiratory rate and a detailed analysis of such biological parameters of a subject can be employed in detecting hypertension, AF, CAD, and other conditions or the early onset thereof. However, these measures are often not employed on a regular basis.

AF is the most common arrhythmia encountered in clinical practice and its paroxysmal nature renders its detection difficult. Without specific therapy, the risk for stroke and congestive heart failure increases significantly. The paroxysmal nature of AF may be present for years before it becomes persistent. This particular property of AF renders its detection difficult and often unsuccessful. Recent trials (see, e.g., Gladstone DJ, Spring M, Dorian P, Panzov V, Thorpe KE, Hall J, et al. "Atrial fibrillation in patients with cryptogenic stroke", The New England journal of medicine 2014; 370: 2467-2477; Sanna T, Diener HC, Passman RS, Di Lazzaro V, Bernstein RA, Morillo CA, et al. "Cryptogenic stroke and underlying atrial fibrillation", N Engl J Med 2014; 370: 2478-2486) support the use of intensified diagnostic strategies to detect AF in selected patients, although the employed methods can be costly or inconvenient. Even with the rapidly increasing knowledge in this field, the relevance of subclinical AF and the temporal correlation between AF and stroke remains controversial and is still being addressed in ongoing trials (see, e.g., Healey JS, Connolly SJ, Gold MR, Israel CW, Van Gelder IC, Capucci A, et al. "Subclinical atrial fibrillation and the risk of stroke", N Engl J Med 2012; 366: 120-129; Ziegler PD, Glotzer TV, Daoud EG, Wyse DG, Singer DE, Ezekowitz MD, et al. "Incidence of newly detected atrial arrhythmias via implantable devices in patients with a history of thromboembolic events", Stroke; 41: 256-260).

The use of smartphones and smart watches in medical practice has received increased attention in the recent past. Suitable devices are equipped with plethysmographic sensors configured to monitor the heart rate. Pulse wave analysis can be employed in order to record and process different biological properties of a patient, based on which certain medical conditions can be determined.

Blood pressure is the pressure exerted by circulating blood upon the walls of blood vessels and is one of the principal vital signs of a person. It is regulated by the nervous and endocrine systems and varies depending on a number of factors including current activity and general health condition of a person. Pathologically low blood pressure is referred to as hypotension, and pathologically high blood pressure is referred to as hypertension. Both pathologies can have different causes and can range from mild to severe, with both acute and chronic forms. Chronic hypertension is a risk factor for many complications, including peripheral vascular disease, heart attack, and stroke. Both hypertension and hypotension are often undetected for longer periods of time because of infrequent monitoring.

Hypertension is generally more common and constitutes the predominant risk factor for a cardiovascular disease and associated health problems including death, higher than those for smoking and diabetes. One major problem with hypertension is that high blood pressure does not necessarily entail pronounced symptoms and that, consequently, there are many people living their lives without realizing that they have elevated or high blood pressure. Measuring and monitoring blood pressure can be done in a number of ways, including at home, as an outpatient, or as an inpatient. However, sporadic and/or infrequent measurements are typically not meaningful enough for effective early detection of hypertension and associated diseases, due to the intervals between measurements often being too long and the measurements being done not often enough.

Medical professionals commonly measure arterial pressure using a sphygmomanometer, which historically used the height of a column of mercury to reflect the circulating pressure, and blood pressure values are typically reported in millimeters of mercury (mm Hg). For each heartbeat, blood pressure varies between systolic and diastolic pressures. Systolic pressure is the peak pressure in the arteries, occurring near the end of a cardiac cycle when the ventricles are contracting. Diastolic pressure is the minimum pressure in the arteries, occurring near the beginning of a cardiac cycle when the ventricles are filled with blood. Typical normal measured values for a resting and healthy adult are 120 mm Hg systolic pressure and 80 mm Hg diastolic pressure (i.e. 120/80 mm Hg).

Systolic and diastolic arterial blood pressures are not static but undergo natural variations from one heartbeat to the next and throughout the day (in a circadian rhythm). Variations occur in response to stress or exercise, changes in nutrition, and disease or associated medication. Blood pressure is one of the four main vital signs, further including body temperature, respiratory rate, and pulse rate, that are routinely monitored by medical professionals and healthcare providers.

Blood pressure can be measured in a noninvasive manner, including palpation, auscultatory or oscillometric methods, continuous noninvasive techniques (CNAP), and based on the pulse wave velocity (PWV) principle. Measuring blood pressure invasively, for example using intravascular cannulae, can produce very accurate measurements, but is much less common due to its invasive nature and is typically restricted to inpatient treatment.

Blood pressure in humans is significantly affected by the elasticity of the vascular system. The elasticity of the vascular system of a person depends on different factors including age, but also on the presence or absence of particular diseases or illnesses. If, for example, the elasticity of the vascular system of a patient decreases due to old age or due to the patient suffering from arteriosclerosis, the blood pressure of the patient increases.

The heart rate (HR) of a subject and the respiratory rate of a subject can also be determined by a physician using known methods for inpatient treatment. Also these measurements are typically taken only at irregular intervals and/or with long periods of time without measurements in between.

The variability of certain biological parameters, such as heart rate, respiration, blood pressure, can serve as an indicator for medical conditions, for example sleep apnea, depression, AF, CAD. The term "variability" can mean a single variability value or measure or a plurality of values indicative of the variability of the respective parameter over time. Any known representations of variabilities are accepted within the scope of the present document.

A. Seeck, W. Rademacher, C. Fischer, J. Haueisen, R. Surber, A. Voss, "Prediction of atrial fibrillation recurrence after cardioversion-Interaction analysis of cardiac autonomic regulation" have found in a study that the assessment of the autonomic regulation by analyzing the coupling of heart rate and systolic blood pressure provides a potential tool for the prediction of arterial fibrillation recurrence after CV and could aid in the adjustment of therapeutic options for patients with arterial fibrillation.

W. Poppe et al., "Eignen sich die Hüllungskurven von Arterienpulswellen für eine Fernbeurteilung psychotischer Krankheitsverläufe?", have found that the envelope of the arterial pulse wave can be indicative of a subject being classified with respect to a particular psychosis and further indicative of a likely progression of a psychosis. This research applies to, for example, the correlation of depression with pulse wave data.

GB 25059890 A discloses a screening procedure and apparatus for identifying cardiac arrhythmia. The values of a sequence of multiple heart pulse intervals are analysed to determine a risk factor whose value enables those at risk of cardiac arrhythmia to be identified. The risk parameter may be calculated from a first measure calculated from the ratio between the variability between successive intervals in the sequence, and the mean interval value, and a second measure relating to a proportion of cases in which a long interval is followed by another long interval in the sequence of heart rate intervals.

Green Geoffrey C. et al.: "Continuous Multiorgan Variability Analysis To Track Severity Of Organ Failure In Critically Ill Patients", Journal Of Critical Care, vol. 28, no. 5, 31 October 2013, evaluates the utility of using continuous heart rate variability and respiratory rate variability monitoring for tracking daily organ dysfunction in critically ill patients and identifying patterns of variability changes during onset of shock and resolution of respiratory failure based on ECG and capnogram data.

Dash S. et al.: "Automatic Real Time Detection Of Atrial Fibrillation", Annals Of Biomedical Engineering, Kluwer Academic Publishers-Plenum Publishers, NE, vol. 37, no. 9, 17 June 2009, describes automatic detection of AF based on the randomness, variability and complexity of heart beat intervals, employing Root Mean Square of Successive Differences (RMSSD) and Shannon Entropy (SE).

WO 2009/152521 discloses methods for non-invasive early screening of a subject for a neurodegenerative disorder, by analyzing one or more parameters associated with the neurodegenerative disorder. The method may include a time domain measure selected from the standard deviation of R-R intervals (SDNN), the standard deviation of the heart rate (SDHR), the root mean square difference of successive RR intervals (RMSSD) and the percentage number of consecutive RR intervals differing by more than 50 msec (pNN50).

Brisinda D. et al.: "Discriminant Analysis Of Heart Rate Variability After Electrical Cardioversion Predicts Atrial Fibrillation Recurrence", International Journal Of Clinical Cardiology, 25 November 2014, describes that cardiac autonomic modulation (CAM) may be pivotal for atrial fibrillation (AF) occurrence and affect early recurrence (ER) after electrical cardioversion (EC) and aims at evaluating whether non-linear (NL) HRV analysis (HRVa) could provide better accuracy in predicting ER.

An aim of the present invention is to provide an apparatus for accurately determining biological parameters of a subject, for example heart rate and respiration and the variability thereof, in a noninvasive manner, easily, and efficiently. It is a further aim to provide an apparatus for determining the biological parameters of a subject and the variabilities thereof with an improved accuracy.

A further aim of the present invention is to provide an apparatus for performing the non-invasive method for determining the blood pressure of a human subject. In particular, the apparatus is a mobile device, and preferably a conventional smart phone provided with a light source and an optical sensor.

**Summary of invention**

According to the invention, there is provided an apparatus for determining a medical condition of a human subject, the apparatus comprising a control unit; and a means for providing pulse wave data representative of a heart beat of the human subject; wherein the control unit is configured to perform the steps of receiving the pulse wave data; selecting a portion of the pulse wave data indicative of a plurality of heart periods; determining a second index indicative of a heart rate variability based on the pulse wave data of the portion of the pulse wave data indicative of a plurality of heart periods; determining a third index indicative of a heart rate variability based on the pulse wave data of the portion of the pulse wave data indicative of a plurality of heart periods, the second index being different from the third index; and determining a medical condition of the subject based on the first and second indexes.

In an aspect, the control unit is configured to further perform the step of determining a first index indicative of a heart rate variability based on the pulse wave data of the portion of the pulse wave data indicative of a plurality of heart periods. Determining the medical condition of the subject is further based on the first index. Determining the first index comprises determining a plurality of respiratory rate intervals based on the pulse wave data of the portion of the pulse wave data indicative of a plurality of heart periods; and determining the first index based on the plurality of respiratory rate intervals.

In a further aspect, determining the first index further comprises determining an average based on the plurality of respiratory rate intervals; and determining the first index based on the average.

In a further aspect, the average is the root mean square of successive difference, optionally wherein determining the root mean square of successive difference based on the plurality of respiratory rate intervals includes normalizing the root mean square of successive difference based on a mean respiratory rate interval determined based on the plurality of respiratory rate intervals.

According to the invention, determining the second index comprises the steps of determining a tachogram indicative of a variability of a plurality of respiratory rate intervals based on the pulse wave data of the portion of the pulse wave data indicative of a plurality of heart periods; determining a frequency distribution of respective respiratory rate intervals of the plurality of respiratory rate intervals; determining an entropy value based on the frequency distribution; and determining the second index based on the entropy value.

In a further aspect, the frequency distribution comprises a histogram indicative of a plurality of probabilities; optionally wherein the entropy value is determined based on $S = -\sum_{i=1} p_i \cdot log_2(p_i)$, wherein pi correspond to the plurality of probabilities; further optionally wherein the histogram has a bin size of 8 ms.

According to the invention, determining the third index comprises the steps of determining a plurality of beat-to-beat intervals (BBI) based on the pulse wave data of the portion of the pulse wave data indicative of a plurality of heart periods; and determining the third index based on the plurality of beat-to-beat intervals.

In a further aspect, determining the third index comprises the steps of determining a Poincare Plot Analysis (PPA) based on the plurality of beat-to-beat intervals, the Poincare Plot Analysis being indicative of a time series fluctuation determined based on a respective relationship of a first beat-to-beat interval (BBIn) and a preceding second beat-to-beat interval (BBIn-1) of the plurality of beat-to-beat intervals; and determining the third index based on the Poincare Plot Analysis.

In a further aspect, determining the third index comprises the steps of determining a standard deviation SD1 of a short-term beat-to-beat interval variability and a standard deviation SD2 of a long-term beat-to-beat interval variability; and determining the third index based on an index SD1/SD2 indicative of a ratio of the standard deviation SD1 to the standard deviation SD2.

In a further aspect, determining the third index comprises determining a plurality of beat-to-beat intervals (BBI) based on the pulse wave data of the portion of the pulse wave data indicative of a plurality of heart periods; and determining the third index based on the plurality of beat-to-beat intervals.

In a further aspect, determining the third index comprises determining a Poincare Plot Analysis (PPA) based on the plurality of beat-to-beat intervals, the Poincare Plot Analysis being indicative of a time series fluctuation determined based on a respective relationship of a first beat-to-beat interval (BBIn) and a preceding second beat-to-beat interval (BBIn-1) of the plurality of beat-to-beat intervals; and determining the third index based on the Poincare Plot Analysis.

In a further aspect, determining the third index comprises determining a standard deviation SD1 of a short-term beat-to-beat interval variability and a standard deviation SD2 of a long-term beat-to-beat interval variability; and determining the third index based on an index SD1/SD2 indicative of a ratio of the standard deviation SD1 to the standard deviation SD2.

In a further aspect, the pulse wave data indicative of a plurality of heart periods relates to a plurality of heart periods in direct succession to one another.

In a further aspect, the portion of the pulse wave data indicative of a plurality of heart periods covers a period of at least 2 minutes; and the steps of determining the second and third indexes is based on substantially all heart beats comprised in the portion of the pulse wave data indicative of a plurality of heart periods.

In a further aspect, the portion of the pulse wave data indicative of a plurality of heart periods covers a period of at least 5 minutes.

In a further aspect, the control unit is further configured to determine, based the pulse wave data of the portion of the pulse wave data indicative of a plurality of heart periods, for each heart period of the plurality of heart periods, whether the respective heart periods is associated with one or more disruptions, and modify the pulse wave data of the portion of the pulse wave data indicative of a plurality of heart periods if the respective heart periods is associated with one or more disruptions so that the respective heart period is no more associated with the one or more disruptions.

In a further, aspect, the one or more disruptions comprise a premature beat.

Advantages of the apparatus for determining the blood pressure include that the blood pressure can be determined with improved accuracy.

## Brief description of drawings

FIG. 1 contains a flow chart of a method for recording pulse wave data in accordance with the present invention, using a mobile device;

FIG. 2A illustrates an exemplary mobile device that can be used in accordance with the method of FIG. 1

FIG. 2B illustrates an interaction of a human subject with the mobile device shown in FIG. 2A;

FIG. 3A illustrates the detection of the respiratory rate in accordance with one embodiment of the invention;

FIG. 3B illustrates how the stiffness index can be determined;

FIG. 3C contains a flow chart of a method for determining blood pressure;

FIG. 3D illustrates the relationship of the heart rate, blood pressure, and respiratory rate, as well as the variabilities thereof, in accordance with one embodiment of the invention;

FIG. 3E contains a flowchart for a method for pulse wave analysis in accordance with the present invention.

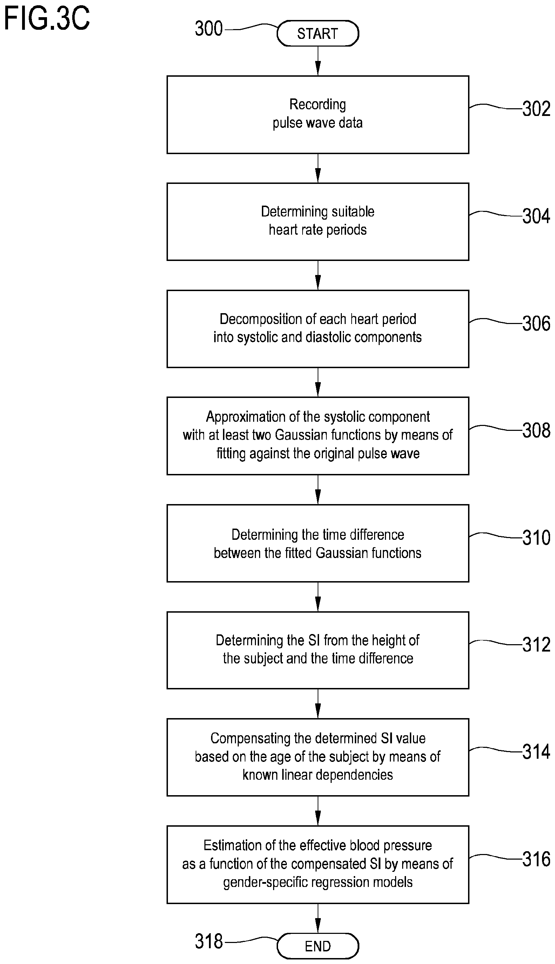

FIG. 3C illustrates the application of the Shannon Entropy in detecting atrial fibrillation in a subject in accordance with one embodiment of the invention;

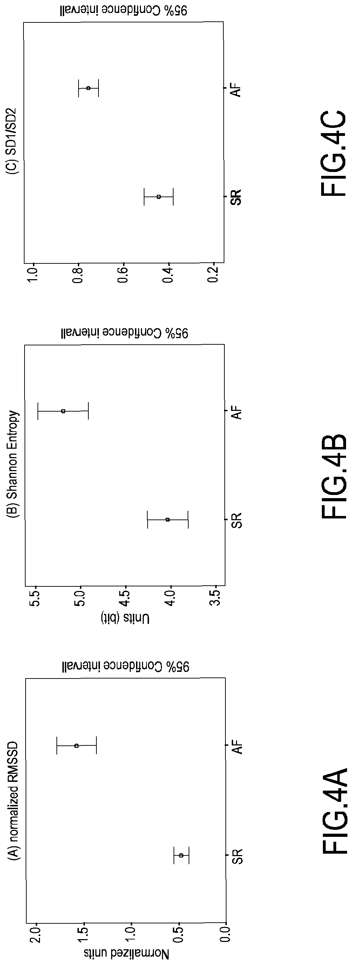

FIG. 4A illustrates the application of RMSSD in detecting AF in a subject in accordance with one embodiment of the invention;

FIG. 4B illustrates the application of the Shannon Entropy in detecting AF in a subject in accordance with one embodiment of the invention;

FIG. 4C illustrates the application of Poincare Plot Analysis in detecting AF in a subject in accordance with one embodiment of the invention; and

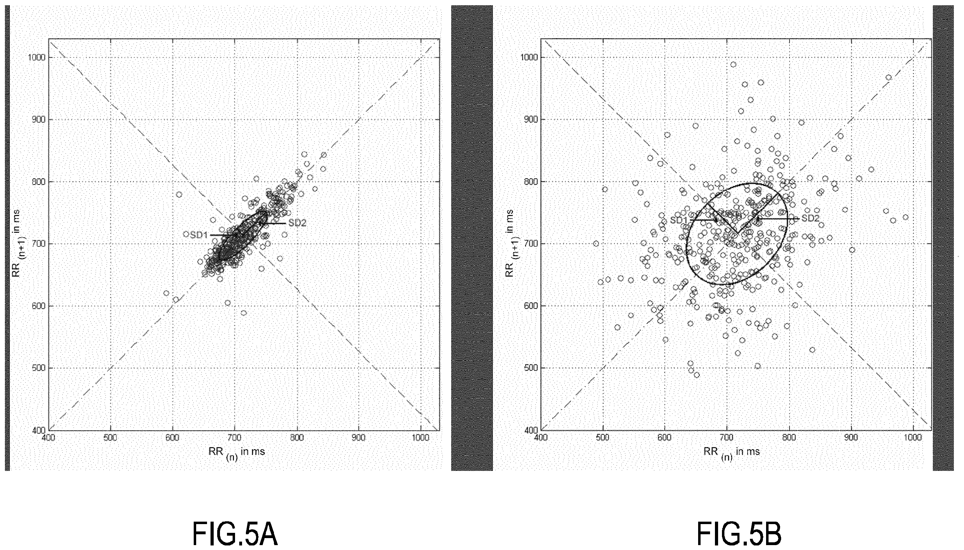

FIGs. 5A and 5B illustrate the application of Poincare Plot Analysis in detecting AF in a subject in accordance with one embodiment of the invention.

## Detailed Description

The elasticity of the vascular system influences the pulse wave of a subject. Based on this effect it has become possible to accurately determine (i.e. in the region of 90% accuracy or more) the blood pressure using an advanced form of photoplethysmography based on specific processing of the pulse wave data. Heart rate and respiratory rate can also be determine based on the pulse wave data of a subject.

The detailed analysis of each of these parameters can form the basis for determining individual conditions of a subject. However, it has been found that, given an accurate representation of the pulse wave data and taking measurements at regular intervals or continuously, the analysis of the heart rate (HR) and the heart rate variability, the blood pressure (BP) and the blood pressure variability, and the respiratory rate and the variability of the respiratory rate can serve to detect a range of medical conditions, such as CAD, AF, sleep apnea, depression and others.

The respiratory rate and the variability thereof can be detected based on an advanced processing of pulse wave data. According to the invention, multiple physiological parameters are simultaneously processed using a novel pulse wave analysis and nonlinear methods for signal analysis. No additional peripheral devices are needed except for a smartphone or smart watch. The apparatus is directed at providing an improved accuracy when differentiating between patients in AF and patients in Sinus Rhythm (SR).

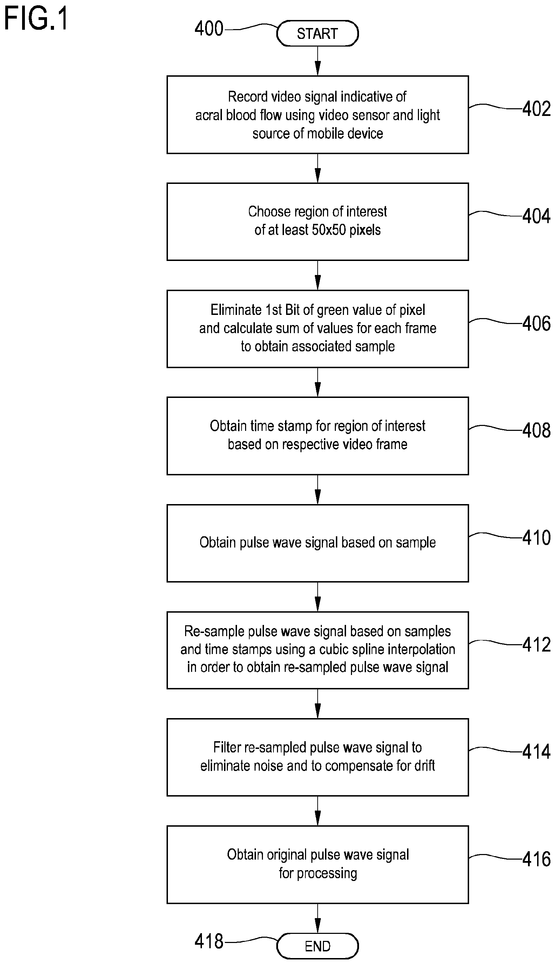

FIG. 1 contains a flow chart of a method 400 for recording pulse wave data in accordance with the present invention, using a mobile device having video recording capabilities. Mobile communication devices, in particular so-called smart phones, have extensive capabilities beyond mere telecommunication. For example, most mobile phones are typically provided with a digital camera capable of capturing still images and video and with a corresponding light source for low-light situations. In general, to record a pulse wave by detecting, with an optical sensor, light emitted from a light source and reflected by a finger of a subject. In one embodiment, pulse wave data is obtained using a common mobile device equipped with a digital camera (e.g. used as an optical sensor) and an LED flashlight (e.g. used as a light source). The light emitted by the light source is reflected and the properties of the light (e.g. intensity, hue, brightness, saturation) are affected (e.g. one or more of the properties are modulated) by the acral blood flow.

In step 402, the subject places their finger on both the light source and the camera of the mobile device such that light emitted from the light source illuminates the acral blood flow and is reflected and detected by the camera. The video signal thus created is recorded and stored in a memory unit of the device. Alternatively, the video signal (e.g. a video stream) can be processed directly, without necessitating storing the pulse wave data in a memory unit.

In step 404, a region of interest (ROI) is selected from the full resolution video stream. This selection can be performed, for example, based on brightness information contained in the video stream. In one embodiment, the ROI is determined in a region of maximum brightness within a video frame, off the center and at a minimum distance from the border. This can ensure that a region is chosen that is sufficiently illuminated (e.g. a region close to the light source). In one embodiment, the ROI has a size of at least 50 x 50 pixels (i.e. 2500 square pixels). Generally, the ROI can have a size ranging from 625 to 10000 square pixels, preferably 900 to 6400 square pixels, more preferably 1600 to 3200 square pixels.

In step 406, for the ROI of each frame of the video stream, a sample $s_i$ is calculated, based on

$$s_i = \sum_{j=0}^{N-1} \sum_{k=0}^{M-1} \frac{p(j \cdot w + k)}{2}$$

with $p$ being the value of the green channel of the pixel located within the ROI at the position $j,k$; N and M being the size of the ROI; and $w$ being the width of the ROI. The division by 2 eliminates the lowest Bit of $p,$ such that noise is effectively reduced. This produces a sample $s_i$ for each captured video frame. In alternative embodiments, a different channel or channels (e.g. red, blue, or a combination of red, green, and/or blue) can be employed instead of the green channel. This may also depend upon the individual device used (e.g. make and model of smartphone, smart watch).

In step 408, a time stamp $t_i$ is generated for each sample $s_i$(more accurately, for each video frame, based on which the sample was calculated) and encoded into the video stream by the video camera.

In step 410, the pulse wave is obtained as a pulse wave signal based on the samples $s_i$ obtained in step 406.

In step 412, a re-sampled pulse wave is obtained by re-sampling the pulse wave from the samples $s_i$ (i.e. as obtained in step 410) based on the associated time stamps obtained in step 408. This is necessary due to technical issues in detecting, generating, and encoding video data, for example resulting in dropped frames or non-constant frame rates. Based on these issues, the samples $s_i$ cannot be obtained at fixed and reliable time intervals. In order to obtain the re-sampled pulse wave, the pulse wave is re-sampled using a cubic spline interpolation and is performed on each polynomial. Here, two subsequent samples are interpolated by a third-degree polynomial. The position (in time) of the samples corresponds to the time stamps. The polynomial $S_i$ for the range $[t_i, t_{i+1}]$ is calculated as follows:

$$S_i = a_i + b_i(t - t_i)^2 + d_i(t - t_i)^3$$

with $i$=1, ..., $n$-1. The process of re-sampling includes incrementing t continuously by 1 ms, corresponding to a sample rate of 1000 Hz. The parameters $a_i$, $b_i$, $c_i$, and $d_i$ have to be set to suitable values. The pulse wave is obtained as the signal S being the result of the re-sampling. In an alternative embodiment, the re-sampling includes incrementing t continuously by 10 ms, corresponding to a sample rate of 100 Hz.

In step 414, the re-sampled pulse wave is filtered to eliminate noise and to compensate for drift. This can be achieved by applying a common bandpass filter (e.g. 0.1 to 10 Hz).

In step 416, the original pulse wave signal is obtained. The original pulse wave can then be processed further, for example as described below with respect to FIG. 3C (see, e.g., steps 304 ff.).

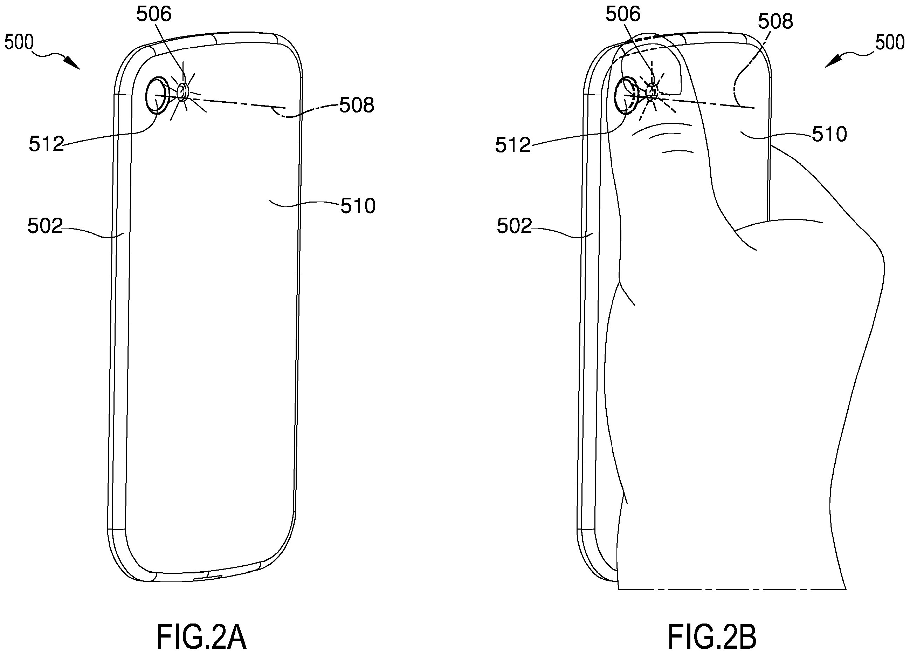

FIG. 2A illustrates an exemplary mobile device that can be used in accordance with the method of FIG. 1. The mobile device 500 has a frame or main body 502 and a device panel 510. In some examples, the device panel 510 can be a back panel of the mobile device 500. The device 500 further has a camera device 512 capable of detecting digital video signals, for example in the form of digital still images and digital video. The camera device 512 is configured to detect video signals representative of objects located generally with a frustum-shaped region along a main detection direction 508. The device 500 further has a light source 506 configured to illuminate any objects located in front of camera device 512, i.e. located within the frustum-shaped region and/or along a main detection direction 508. The light source 506 can be configured to provide both a single flash of light and a continuous light beam, depending on a mode of operation. When recording video, the light source typically provides a continuous light beam. An object placed within the view of camera device 512 will reflect light emitted from light source 506 so that the reflected light can be detected by camera device 512. Mobile device 500 further comprises a control unit (e.g. a CPU, micro processor, SoC; not shown) coupled to other components, such as camera device 512, light source 506, a memory unit, a user interface, input means, an audio unit, a video unit, a display, and other.

FIG. 2B illustrates an interaction of a human subject with the mobile device shown in FIG. 2A. In order to take a measurement, the subject places a finger (e.g. a thumb) on mobile device 500, covering both the camera device 512 and the light source 506. The individual configuration of the mobile device (e.g. a position of camera device 512 and light source 506 or the distance in between) is of secondary relevance, as long as it is physically possible to cover both the camera device 512 and the light source 506 with a suitable extremity (e.g. finger, thumb, ear). In this respect, any extremity suitable for (acral) measurement can be used in accordance with the present invention. In general, any body part that is associated with pulsating blood flow can be used in accordance with the present invention, as long as a meaningful signal indicative of the blood flow can be detected via the body part. In some embodiments, the control unit of mobile device 500 will process signals provided by camera device 512 and detect, based on the signals provided, that one or more parameters indicative of video quality (e.g. brightness, contrast, focus) are outside of preferred operating ranges due to the low-light and/or close-proximity situation created by the placement of the thumb directly onto camera device 512. The control unit may then provide control signals to one or more components, for example to light source 506, in order to make adjustments to the parameters (e.g. activating light source 506 in order to compensate for low light).

Upon placement of the suitable extremity (here, e.g., the thumb of the subject), the measurement is initiated by activating the light source 506 to emit a continuous light beam of sufficient intensity, such that acral blood flow is illuminated. At substantially the same time, camera device 512 is activated and the light reflected by the acral blood flow is detected by camera device 512. Both activating the light source 506 and activating the camera device 512 can be achieved by corresponding program code executed by the control unit comprised in device 500. The activation can be triggered manually, for example by selecting a corresponding function on a user interface of device 500, or automatically, for example triggered by a sensor (e.g. a proximity sensor, an optical sensor), a timer, voice recognition, or other (input means). In one example, the signal of the sensor is continuously processed to check for the presence of a suitable signal. Video data is then recorded or transmitted for further processing for a predetermined period of time, typically ranging from several seconds to 2 minutes. In some embodiments, the time period is not predetermined, but determined as the recording/transmitting is ongoing, in that a quality measure is calculated from the recorded/transmitted data and the recording/transmitting is performed until a sufficient number of heart periods (e.g. 10-30) of sufficient quality (e.g. similarity; see in further detail below) has been recorded/transmitted. Completion of the recording/transmitting can be indicated to the subject, for example, by an acoustic and/or optical signal emitted by an audio and/or video component of device 500.

It is noted that other embodiments employ the same or different sensors and/or devices. For example, smart watches having a corresponding light source/sensor assembly as described above with respect to FIGs. 2A and 2B, can be used as well. These devices have an advantage in that the sensor is kept in close proximity to the body (here: wrist) of a subject, thereby facilitating continuous measurements and/or measurements of arbitrary duration and at arbitrary time points, without interaction of a subject (e.g. during sleep or other activities). It is noted that the above concepts apply to a range of sensors and are not limited to a particular or otherwise specific embodiment of source/sensor hardware.

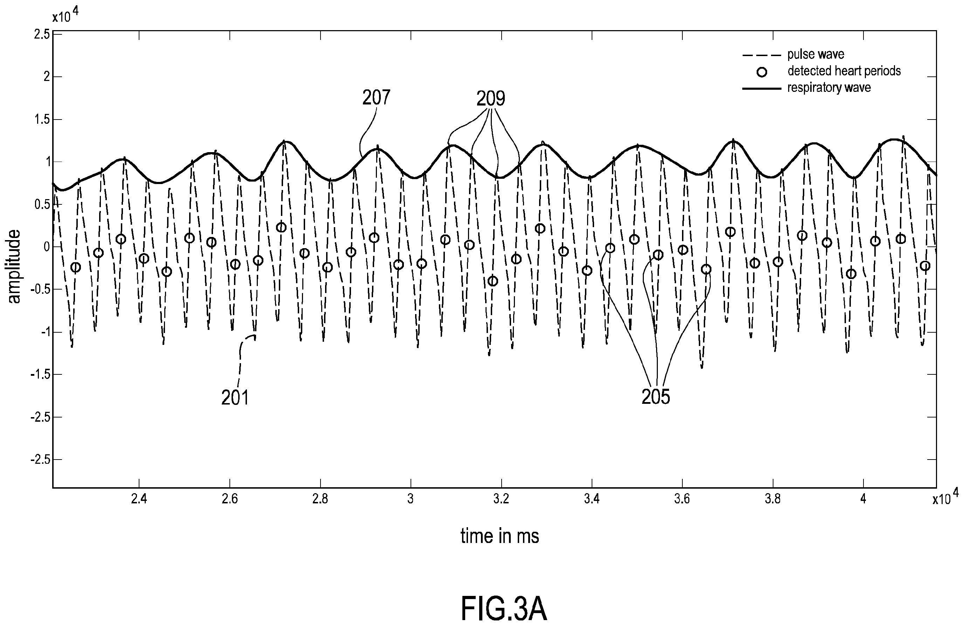

FIG. 3A illustrates the detection of the respiratory rate in accordance with an embodiment of the invention. FIG 3A shows, on the vertical axis, the amplitude of a detected pulse wave 201 over time (see horizontal axis). The pulse wave 201 exhibits an amplitude of between approximately -1 and 1, and the instances of rising edges are registered as detected heart periods 205. Further, a signal 207 indicative of the respiration of the subject is detected based on the maxima 209 of the pulse wave 201.

In order to obtain the signal, the maxima 209 of the pulse wave are sampled using a cubic spline interpolation similar to the re-sampling of the pulse-wave described with respect to FIG. 1 above. Here, two subsequent samples are interpolated by a third-degree polynomial. The position (in time) of the samples corresponds to the time stamps. The polynomial $R_i$ for the range $[t_i, t_{i+1}]$ is calculated as follows:

$$R_i = a_i + b_i(t - t_i)^2 + d_i(t - t_i)^3$$

with $i = 1, ..., n\text{-}1$. The process of re-sampling includes incrementing t continuously by 1 ms, corresponding to a sample rate of 1000 Hz. In an alternative embodiment, the re-sampling includes incrementing t continuously by 10 ms, corresponding to a sample rate of 100 Hz. The parameters $a_i$, $b_i$, $c_i$, and $d_i$ have to be set to suitable values. The pulse wave is obtained as the respiration R, i.e. signal 207, being the result of the sampling. The variation of the respiration rate is then determined based on signal 207 by known methods, for example by detecting a series of maxima of signal 207 and determining a time difference for each pair of subsequent maxima.

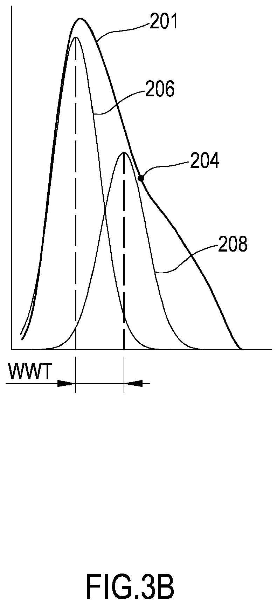

FIG. 3B illustrates how the stiffness index can be determined. The diagram in FIG. 3B shows a pulse wave signal 201 over time t as well as corresponding wave components 206 and 208 of the original pulse wave and the wave reflected mainly by the aortic bifurcation. FIG. 3B also shows an inflection point 204. It is noted that a simple partitioning based on the inflection points, as commonly known in the art, does not necessarily correspond to the actual physiological wave components because of the reasons set forth in the previous paragraph. In contrast, in accordance with the present invention, the actual original pulse wave and the wave reflected by the aortic bifurcation are determined by approximation of the graph with Gaussian functions, by which the two component waves $w_{original}$ 206 and $w_{reflected}$ 208 can be obtained with very high accuracy. Here, the time difference is determined as the time difference between the component waves $w_{original}$ and $w_{reflected}$ as opposed to the time difference between two maxima of the graph. This facilitates determining, instead of the commonly known peak-to-peak time (PPT), a wave-to-wave time (WWT), which corresponds to the actual time difference between the original pulse wave and the reflected pulse wave with a substantially higher level of accuracy. This, in turn, facilitates a more accurate calculation of the SI and, thus, leads to an improved correlation with the blood pressure.

FIG. 3C contains a flow chart of a method 300 for determining blood pressure. In step 302, pulse wave data is recorded. The detection of pulse waves and the recording of data indicative of the detected pulse wave can be performed in any way known in the art. For example, classic photoplethysmography. One example of detection and recording of pulse wave data has been described above with respect to FIG. 1.

In step 304 suitable heart periods are determined. As described above, heart periods vary depending on a number of factors and can exhibit benign (e.g. non-pathological) irregularities, for example caused by stress or anxiety, or consumption of stimulants such as caffeine, nicotine, or alcohol. In order to establish a sound basis for further processing of pulse wave data, suitable heart periods are selected from a longer recording of pule wave data. In the first embodiment, 5 to 30 heart periods are selected from a pulse wave recording of 5 seconds up to 2 minutes in length, provided that all selected heart periods have a similarity to each other of at least 0.8 and are all contained in a single recording segment (i.e. are successive to each other). In other embodiments, a greater or smaller number of successive heart periods may be used, for example 3 to 10 or 20 to 50 heart periods. Further, the recording of pulse wave data can have a different length, for example ranging from 5 to 10 seconds up to 10 to 30 minutes.

In step 306, each heart period is decomposed or partitioned into a systolic and a diastolic component. This is achieved by determining the maximum of the second order derivative of the pulse wave, located at most 350 ms after the systolic maximum. Typically, the maximum of the second order derivative of the pulse wave is located between 250 ms and 350 ms after the systolic maximum. Determining the maximum of the second order derivative is restricted to the above-defined time window in order to take into account the expulsion time of the heart and in order to avoid erroneous detection.

In step 308, an approximation is performed in which the systolic component is approximated by fitting at least two Gaussian functions to the original pulse wave:

$$F(a,b,c,d,f) = \sum_{i=1}^{N} \left( S_i - \left( a \cdot e^{-\frac{1}{2}\left(\frac{t-b}{c}\right)^2} + d \cdot e^{-\frac{1}{2}\left(\frac{t-f}{c}\right)^2} \right) \right)^2 \overset{!}{=} min$$

with *a, b, c,* and *d* being determined using non-linear optimization. In one embodiment, the two Gaussian functions are fitted to the original pulse wave using the Levenberg-Marquardt algorithm. In this approximation step, the first Gaussian function corresponds to the original pulse wave and the second Gaussian function corresponds to the wave reflected at the aortic bifurcation, whereas the amplitude of the first Gaussian function must be greater or equal to the amplitude of the first Gaussian function, and both functions must exhibit an identical standard deviation a.

In step 310, the time difference between the two Gaussian functions is calculated as the wave-to-wave time WWT. For example, the WWT can be calculated as the time difference between the base points of the two Gaussian functions. Alternatively, the WWT can be calculated as the time difference between the maxima of the two Gaussian functions. In order to generate an overall or averaged $WWT_a$, the median value over 5 to 30 (or any desired number of) heart periods is calculated. This can effectively reduce the impact of outliers.

In step 312, the stiffness index SI is calculated based on the subject height h (in m) and the averaged $WWT_a$ (in s) as:

$$SI = \frac{h}{WWT_a}$$

In step 314, the SI value calculated in step 312 is adjusted in order to compensate for the age of the subject. As described above, the elasticity of a person's vascular system decreases with increasing age, so that the average healthy person at an age of 20 necessarily exhibits a lower SI than the average healthy person at an age of 40 or 60. Therefore, the SI is normalized in step 314 in order to achieve comparable results. In the first embodiment, the SI is normalized in order to obtain an age-independent or adjusted SI.

In step 316, the adjusted SI is estimated based on a gender-specific regression model. The gender-specific regression models are the result of proprietary clinical studies and define the estimated blood pressure of a subject as a function of gender and the adjusted SI. In one example, a male person exhibiting an adjusted SI of 10 may have an estimated systolic blood pressure of 180 mm Hg. Clinical studies were conducted in order to determine how the adjusted SI relates to the actual blood pressure depending on the gender of a subject. It has been found that, with male subjects, an adjusted SI of about 10 m/s corresponds to a blood pressure of about 170 mm Hg, and an adjusted SI of about 8 m/s corresponds to a blood pressure of about 150 mm Hg. With female subjects, an adjusted SI of about 10 m/s corresponds to a blood pressure of about 165 mm Hg, and an adjusted SI of about 8 m/s corresponds to a blood pressure of about 155 mm Hg.

In an alternative embodiment, a more comprehensive regression model is applied. In this alternative embodiment, steps 302 to 314 are performed identical to what is described above. In step 316 of the alternative embodiment, however, additional parameters are applied in order to achieve an even higher correlation to the actual blood pressure value. Here, the adjusted SI is estimated based on an alternative regression model that factors in: the gender of the subject (i.e. male or female), an index value $I_f$ indicative of the physique of the subject (e.g. the body mass index (BMI) of the person), and an index value $C_t$ indicative of a tobacco consumption of the subject (e.g. whether or not the subject is a smoker).

With respect to the index value $C_t$ indicative of a tobacco consumption of the subject it is noted that in some embodiments only the current status of a subject is determined, namely whether the subject is currently an active smoker. Studies have shown that a relatively short period of not smoking has an impact on blood pressure in a subject, even if the subject had smoked for an extensive period of time. This effect and related effects can be taken into account by determining the current status of a subject in this manner. In other embodiments the history of the subject can also be taken into account. This can be done by determining a period or periods in which the subject was an active smoker and determining the amount of tobacco consumed in these periods (e.g. number of cigarettes per day). In this manner an individual profile detailing the consumption of tobacco by a subject can be generated and introduced into the regression model. It is noted that long-term tobacco consumption can have multiple effects on the vascular system of a subject, for example regarding stiffness of the blood vessels. Some or all of these effects can be long-term effects that do not disappear during a short period of non-smoking.

One specific alternative regression model, which is also the result of proprietary clinical studies, defines the estimated blood pressure of a subject as a function of the adjusted SI, the gender of the subject (a value of 1 being indicative of a male subject, a value of 2 being indicative of a female subject), the BMI of the subject (the BMI value being calculated based on the height and weight of the subject), and the fact that the subject is a smoker or not (a value of 0 being indicative of the subject not being a smoker, and a value of 1 being indicative of the subject being a smoker).

The BMI can be calculated based on $BMI = \frac{mass}{height^2}$, where mass is the weight of the subject in kilograms (kg) and where height is the height of the subject in meters (m). The specific alternative regression model is based on the formula: $BP_{sys} = 139.611 - 19.450 \cdot g - 0.820 \cdot age + 0.968 \cdot I_f + 5.394 \cdot C_t + 2.759 \cdot SI$.

The following table provides further details on the coefficients used in the alternative regression model:

Coefficients (dep. var: $BP_{sys}$)

| | Model | Non Standardized Coefficients | | Standardized Coefficients | t | Sig. |
|---|---|---|---|---|---|---|
| | | B | Standard Error | Beta | | |
| 1 | (Constant) | 139.611 | 40.618 | | 3.437 | 0.004 |
| | Sex (1=male, 2=female) | -19,450 | 7.278 | -0.568 | -2.673 | 0.019 |
| | Age | -0.820 | 0.478 | -0.318 | -1.717 | 0.110 |
| | If (BMI) | 0.968 | 1.513 | 0.140 | 0.639 | 0.534 |
| | Ct (Smoker) | 5.394 | 6.830 | 0.144 | 0.790 | 0.444 |
| | SI | 2.759 | 2.286 | 0.228 | 1.207 | 0.249 |

It is noted that the term "physique" of the subject refers to the size, stature, figure, or physique in terms of the absence or presence (and the degree) of adiposity of the subject, i.e. whether the person is overweight or not. Apart from the BMI as described above, there are a number of known methods and/or concepts for quantifying a degree of adiposity in a subject. Examples include, but are not limited to: measuring the percentage of body fat (e.g. by bioelectric impedance analysis, by caliper measurements, or any other known method for determining the percentage of body fat), calculating the waist-to-height ratio, and calculating the waist-to-hip ratio. Bioelectric impedance analysis, for example, can be integrated into household appliances like scales, so that the percentage of body fat can be measured during regular or daily activities, such as stepping on the scale to be weighed. Bioelectric impedance analysis may not be applicable to all subjects due to their individual medical condition, for example when a pace maker or other implant is in place, and/or may not provide the most accurate measurements of body fat percentage. Caliper measurements can be made by a physician or by the subject him/herself by measuring the thickness of a skin fold in order to deduce the body fat percentage. The measurements are typically performed at three or seven different body parts, depending on the method used. Caliper measurements can provide acceptable results but typically cannot accurately measure the percentage of body fat present in and around organs.

It is noted that the alternative regression function described above does not require the use of the BMI in particular, but is, in principle, adaptable to any quantification of a degree of adiposity in a subject. If a measure of a subject's physique other than the subject's BMI is used, a corresponding conversion factor needs to be introduced into the specific formula described above, in order to map the measure to the BMI (or vice versa).

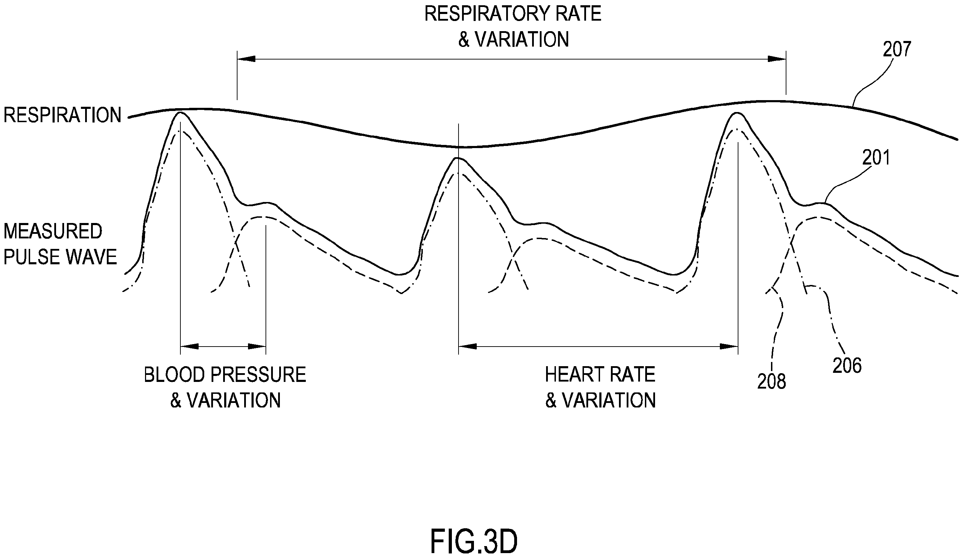

Blood pressure variability is now determined based on a plurality of blood pressure values taken from a subject in the manner described above. Typically, determining blood pressure variability is performed over a period of 2 to 5 minutes, or alternatively, over a number of 120 to 300 heart periods, in order to obtain a representative sample. In other embodiments, however, the determining of blood pressure and blood pressure variability can be performed in a continuous manner, for example using a sliding window of 2 to 5 minutes (or 120 to 300 heart periods).FIG. 3D illustrates the relationship of the heart rate, blood pressure, and respiratory rate, as well as the variability thereof, in accordance with an embodiment of the invention. FIG. 3B shows a combination of a number of signals determined based on the pulse wave 201. Here, the respiratory rate and the variation thereof are shown based on signal 207. Further, the blood pressure and variation thereof are shown based on pulse wave 201 and the components 206 and 208 thereof, as described above and as shown in FIG. 1. The heart rate and variation thereof is also shown based on pulse wave 201.

Based on an analysis of the heart rate (HR) and the heart rate variability, the blood pressure (BP) and the blood pressure variability, and the respiratory rate and/or the variability of the respiratory rate, all obtained based on the pulse wave 201 and exhibiting an accuracy previously not obtainable, a range of medical conditions, such as CAD, AF, sleep apnea, depression and others.

Based on the data obtained, AF can be detected by analyzing the interaction between heart rate and blood pressure using nonlinear interaction dynamics, for example joint symbolic dynamics (JSD) and segmented Poincare plot analysis (SPPA). SPPA can be applied to analyze the interaction between two time series - here heart rate and blood pressure. Introducing a parameter set of two indices, one derived from JSD and one from SPPA, the linear discriminant function analysis revealed an overall accuracy of 89% (sensitivity 91.7%, specificity 86.7%) for the classification between patients with stable sinus rhythm (group SR, n = 15) and with AF recurrence (group REZ, n = 12). The coupling of heart rate and systolic blood pressure provides a potential tool for the prediction of AF recurrence after CV and could aid in the adjustment of therapeutic options for patients with AF.

In a similar manner, depression can be detected by analyzing the relationship between respiration and heart rate and by detecting that respiration and heart rate are not in sync and/or the heart rate does not change upon substantial variation of the respiratory rate. Likewise, sleep apnea can be detected using the above-described mechanisms by analyzing the respiratory rate, typically showing an unusually high variation, and by analyzing the heart rate, typically slowing down during periods of sleep apnea.

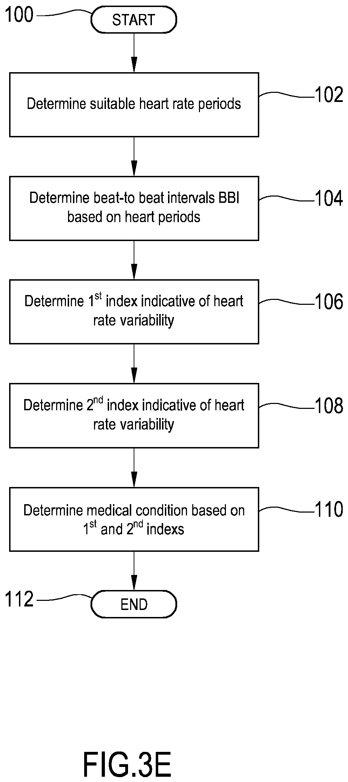

FIG. 3E contains a flowchart for a method 100 for pulse wave analysis in accordance with the present invention. At step 102, the pulse wave signal is acquired as set forth above with respect to FIG. 1 and suitable heart periods are selected. Typically, the pulse wave signal is acquired for a time period of at least 2 minutes, preferably at least 5 minutes.

At step 104, a combination of morphology and frequency analysis of the pulse wave is applied to detect all Beat-to-Beat-Intervals (BBI). The applied algorithm yields an improved correlation of r > 0.99, compared to RR-intervals (RRi) from standard ECG recordings, which were done in comparison. From the extracted BBI time series, several indices representing the variability of heart rhythm can be calculated and analyzed regarding their ability to discriminate between AF and SR. For the analysis, premature beats and other disruptions can be eliminated and corresponding points on the BBI time series can be replaced, using an algorithm for adaptive variance estimation. The impact of ectopy on variability indices is rather low. However, even in groups exhibiting a minor number of ectopic beats (e.g., less than 5%), filtering of the tachogram can further reduce the impact of ectopy.

At steps 106 and 108 first and second indexes are determined in accordance with what is described with respect to FIGs. 4A, 4B, and 4C below. In one embodiment, the first index is a root mean square of successive difference (RMSSD) of RR intervals and the second index is an SD1/SD2 index. In other embodiments, other combinations, for example including an index determined based on the Shannon Entropy, can be employed.

At step 110 the medical condition of the subject is determined, based on the first and second indexes. The method 100 ends at step 112.

FIG. 4A illustrates the application of RMSSD in detecting AF in a subject in accordance with one embodiment of the invention. The RMSSD is a standard index from heart rate variability (HRV) analysis to quantify beat-to-beat alterations. In order to adjust for the effect of heart rate on the RR variability the RMSSD value is normalized to the mean RR interval value. Since in AF the variability is distinctly higher than in SR, normalized RMSSD is expected to be higher in patients with AF. In a first comparative embodiment, normalized RMSSD and Shannon Entropy (ShE) were combined. Both indices were extracted from the pulse wave tachogram. Sensitivity, specificity and accuracy were calculated for each of these indices separately and for the combination. For the discrimination between AF and SR based on a two minute pulse wave recording the ShE yielded a sensitivity and specificity of 85% and 95% respectively, applying a cut-off value of 4.9 (see FIG. 4B). This translates into 34/40 patients classified correctly and 2/40 patients classified incorrectly as AF.

FIG. 4B illustrates the application of the Shannon Entropy in detecting AF in a subject in accordance with one embodiment of the invention. Shannon Entropy (ShE) is a statistical method to quantify uncertainty for a random variable and is expected to be higher in patients with AF since the pulse in these circumstances exhibits greater RR interval irregularity compared to pulses recorded from patients with SR.

Based on the pulse wave, a tachogram is determined, which is indicative of the variations in respiratory rate over time. From the tachogram, a histogram is generated, which represents the frequency distribution of the respiratory rate variations. In one embodiment, the histogram has a bin size of 8ms, which means that the frequency distribution is based on a discrete time scale divided into 8ms slots. Each respiratory variation (i.e. between two maxima of signal 207) is sorted into the respective bin. The probabilities represented by the histogram are then used as input for calculating the Shannon Entropy as

$$S = -\sum_{i=1} p_i \cdot log_2(p_i) \; .$$

The result is a bit value, which determines whether or not a subject belongs to a group of healthy patients or not, whereas a threshold value of 4.9 bits is used:

$$AFib = \begin{cases} S \geq 4.9 \; bit, \; then \; \boldsymbol{yes} \\ otherwise \; \boldsymbol{no} \end{cases}$$

It is noted that the above is one example to determining an entropy value for the respiratory rate variations. Other known methods can be used in a similar manner, by simply adapting the threshold value according to the method and calculation used. FIG. 4C illustrates the threshold value of 4.9, clearly distinguishing between subjects showing AF (right) and subjects showing SR (left). One advantage of determining a frequency distribution in the manner described is that the entropy measure is independent from a resting heart rate of the subject. The above is, thus, equally applicable to subjects of all age groups, for example children as well as the elderly, despite of substantial differences in their respective resting heart rates.

In a second comparative embodiment, a filter was applied to the pulse wave tachogram to eliminate premature beats and other disruptions as described above. This improved the applicability of the method and allowed patients with premature beats to be successfully separated from patients with AF. The application of the tachogram filter improved sensitivity to 87.5% while specificity remained stable at 95% using the index normalized RMSSD with a cut-off at 0.09. This translates into 35/40 patients classified correctly and 2/40 patients classified incorrectly as AF.

FIG. 4C illustrates the application of Poincare Plot Analysis (PPA) in detecting AF in a subject in accordance with one embodiment of the invention. In a third comparative embodiment, an additional index SD1/SD2 that was extracted from a Poincare Plot of a five minute recording was tested. SD1/SD2, normalized RMSSD and Shannon Entropy were calculated from a filtered tachogram. Sensitivity, specificity and accuracy were then calculated for each method separately and for the combination. By prolonging the recording time from two to five minutes, and combining the index SD1/SD2 and normalized RMSSD, sensitivity and specificity increased to 95% with an area under the curve of 0.93 (see FIGs. 5A and 5B). The cut-off for classification as AF was a normalized RMSSD > 0.043 and a SD1/SD2 > 0.6. This translates into 38/40 patients classified correctly and 2/40 patients classified incorrectly as AF.

It was found that the highest sensitivity and specificity was achieved using the combination of the indices normalized RMSSD and SD1/SD2 with the tachogram filter (see third comparative embodiment) in combination with prolonging the analyzed interval from two to five minutes. Consequently, a sensitivity and specificity of 95% was achieved.

The results are based on a group of eighty patients included in a study (AF 40pts, SR at time of examination 40pts). Patients in the AF group had a mean age of 80 years (SD±8), patients in the SR group 75 years (SD±7). Male to female ratio was 2.4 in the AF group and 2.5 in the SR group. The average RR interval was higher in the AF group. (AF 887±120ms and SR 784±144ms, p=0,0004). The results of the comparative embodiments are shown in the following table:

TABLE 1

|  | SR (mean±SD) | AF (mean±SD) | *p* value | AUC | Sensitivity | Specificity |
|---|---|---|---|---|---|---|
| **Method 1** | | | | | | |
| **nRMSSD** | 0.103±0.093 | 0.298±0.121 | <0.001 | 0.892 | 50% | 95% |
| **ShE** | 3.858±0.711 | 5.350±0.825 | <0.001 | 0.912 | 85% | 95% |
| **nRMSSD + ShE** | - | - | - | 0.917 | 82.5% | 95% |
| **Method 2** | | | | | | |
| **nRMSSD** | 0.034±0.026 | 0.146±0.067 | <0.001 | 0.938 | 87.5% | 95% |
| **ShE** | 3.710±0.643 | 5.007±0.790 | <0.001 | 0.911 | 77.5% | 95% |
| **nRMSSD + ShE** | - | - | - | 0.926 | 87.5% | 95% |
| **Method 3** | | | | | | |
| **nRMSSD** | 0.039±0.026 | 0.154±0.070 | <0.001 | 0.942 | 77.5% | 95% |
| **ShE** | 4.030±0.697 | 5.187±0.885 | <0.001 | 0.872 | 57.5% | 95% |
| **SD1/SD2** | 0.447±0.202 | 0.757±0.141 | <0.001 | 0.903 | 77.5% | 90% |
| **nRMSSD + ShE** | - | - | - | 0.966 | 80% | 95% |
| **ShE+ SD1/SD2** | - | - | - | 0.959 | 50% | 95% |
| **nRMSSD + SD1/SD2** | - | - | - | 0.931 | 95% | 95% |

FIGs. 5A and 5B illustrate the application of Poincare Plot Analysis (PPA) in detecting AF in a subject in accordance with one embodiment of the invention. PPA provides a visual tool to characterize the complex nature of time series fluctuations where $BBI_r$ is plotted against $BBI_{n-1}$. The Poincare plot usually displays an elongated cloud of points oriented along the diagonal of the coordinate system. An ellipse is fitted to the cloud of points to characterize its shape. The index SD1/SD2 represents the ratio of the standard deviation of short-term BBI variability (axis vertical to the line of identity, SD1) to the standard deviation of the long-term BBI variability (axis along the line of identity, SD2). The index shown was extracted from five minutes recordings to ensure the formation of the ellipse.

While the invention has been described in connection with what is presently considered to be the most practical and preferred embodiments, it is to be understood that the invention is not to be limited to the disclosed embodiments. The invention is defined by the appended claims.

## Claims

1. An apparatus for determining a medical condition of a human subject, the apparatus comprising:

   a control unit; and
   a means for providing pulse wave data representative of a heart beat of the human subject; wherein
   the control unit is configured to perform the steps of:

   receiving the pulse wave data;
   selecting a portion of the pulse wave data indicative of a plurality of heart periods;
   determining a second and a third index, each of the second and third indexes being based on the pulse wave data of the portion of the pulse wave data indicative of a plurality of heart periods, wherein the third index is different from the second index; and
   determining a medical condition of the subject based on the second and third indexes; wherein determining the second index comprises:

   determining a tachogram indicative of a variability of a plurality of respiratory rate intervals based on the pulse wave data of the portion of the pulse wave data indicative of a plurality of heart periods,

determining a frequency distribution of respective respiratory rate intervals of the plurality of respiratory rate intervals,
determining an entropy value based on the frequency distribution, and
determining the second index based on the entropy value; and

wherein
determining the third index comprises:

determining a plurality of beat-to-beat intervals (BBI) based on the pulse wave data of the portion of the pulse wave data indicative of a plurality of heart periods, and
determining the third index based on the plurality of beat-to-beat intervals.

2. The apparatus according to the preceding claim, further comprising determining a first index, the first index being different from the second and third indexes; wherein

determining the first index comprises:

determining a plurality of RR intervals based on the pulse wave data of the portion of the pulse wave data indicative of a plurality of heart periods, and
determining the first index based on the plurality of RR intervals;

and wherein
determining the medical condition of the subject is further based on the first index.

3. The apparatus according to the preceding claim, wherein determining the first index further comprises:

determining an average based on the plurality of RR intervals; and
determining the first index based on the average.

4. The apparatus according to the preceding claim, wherein the average is the root mean square of successive difference, optionally wherein determining the root mean square of successive difference based on the plurality of respiratory rate intervals includes normalizing the root mean square of successive difference based on a mean RR interval determined based on the plurality of RR intervals.

5. The apparatus according to any one of the preceding claims, wherein the frequency distribution comprises a histogram indicative of a plurality of probabilities; optionally wherein the entropy value is determined based on:

$$S = -\sum_{i=1} p_i \cdot log_2(p_i),$$

wherein $p_i$ correspond to the plurality of probabilities; further optionally wherein the histogram has a bin size of 8 ms.

6. The apparatus according to any one of the preceding claims, wherein determining the third index comprises:

determining a Poincare Plot Analysis (PPA) based on the plurality of beat-to-beat intervals, the Poincare Plot Analysis being indicative of a time series fluctuation determined based on a respective relationship of a first beat-to-beat interval ($BBI_r$) and a preceding second beat-to-beat interval ($BBI_{n-1}$) of the plurality of beat-to-beat intervals; and
determining the second index based on the Poincare Plot Analysis.

7. The apparatus according to the preceding claim, wherein determining the third index comprises:

determining a standard deviation SD1 of a short-term beat-to-beat interval variability and a standard deviation SD2 of a long-term beat-to-beat interval variability; and
determining the second index based on an index SD1/SD2 indicative of a ratio of the standard deviation SD1 to the standard deviation SD2.

8. The apparatus according to any one of the preceding claims, wherein the pulse wave data indicative of a plurality of heart periods relates to a plurality of heart periods in direct succession to one another.

9. The apparatus according to any one of the preceding claims, wherein the portion of the pulse wave data indicative of a plurality of heart periods covers a period of at least 2 minutes; and
determining the second and third indexes, and optionally the first index, is based on substantially all heart beats comprised in the portion of the pulse wave data indicative of a plurality of heart periods.

10. The apparatus according to the preceding claim, wherein the portion of the pulse wave data indicative of a plurality of heart periods covers a period of at least 5 minutes.

11. The apparatus according to any one of the preceding claims, wherein the control unit is further configured to:

determine, based the pulse wave data of the portion of the pulse wave data indicative of a plurality of heart periods, for each heart period of the plurality of heart periods, whether the respective heart period is associated with one or more disruptions, and
modify the pulse wave data of the portion of the pulse wave data indicative of a plurality of heart periods if the respective heart period is associated with one or more disruptions so that the respective heart period is no more associated with the one or more disruptions.

12. The apparatus according to the preceding claim, wherein the one or more disruptions comprise a premature beat.


**Patentansprüche**

1. Vorrichtung zur Bestimmung einer Krankheit eines menschlichen Subjekts, wobei die Vorrichtung umfasst:

eine Steuereinheit; und
ein Mittel zum Bereitstellen von Pulswellendaten, die für einen Herzschlag des menschlichen Subjekts repräsentativ sind; wobei
die Steuereinheit dazu ausgelegt ist, die folgenden Schritte durchzuführen:

Empfangen der Pulswellendaten;
Auswählen eines Teils der Pulswellendaten, der für eine Vielzahl von Herzperioden bezeichnend ist;
Bestimmen eines zweiten und eines dritten Indexes, wobei der zweite und dritte Index jeweils auf den Pulswellendaten des Teils der Pulswellendaten beruhen, der für eine Vielzahl von Herzperioden bezeichnend ist, wobei der dritte Index vom zweiten Index verschieden ist;
und
Bestimmen einer Krankheit des Subjekts auf der Grundlage des zweiten und dritten Indexes; wobei
Bestimmen des zweiten Indexes umfasst:

Bestimmen eines Tachogramms, das für eine Variabilität einer Vielzahl von Atemfrequenzintervallen bezeichnend ist, auf der Grundlage der Pulswellendaten des Teils der Pulswellendaten, der für eine Vielzahl von Herzperioden bezeichnend ist,
Bestimmen einer Frequenzverteilung der jeweiligen Atemfrequenzintervalle der Vielzahl von Atemfrequenzintervallen,
Bestimmen eines Entropiewerts auf der Grundlage der Frequenzverteilung und
Bestimmen des zweiten Indexes auf der Grundlage des Entropiewerts; und

wobei
Bestimmen des dritten Indexes umfasst:

Bestimmen einer Vielzahl von Herzschlagintervallen (BBI) auf der Grundlage der Pulswellendaten des Teils der Pulswellendaten, der für eine Vielzahl von Herzperioden bezeichnend ist, und
Bestimmen des dritten Indexes auf der Grundlage der Vielzahl von Herzschlagintervallen.

2. Vorrichtung nach dem vorhergehenden Anspruch, ferner umfassend Bestimmen eines ersten Indexes, wobei der erste Index vom zweiten und dritten Index verschieden ist; wobei
Bestimmen des ersten Indexes umfasst:

Bestimmen einer Vielzahl von AF-Intervallen auf der Grundlage der Pulswellendaten des Teils der Pulswellendaten, der für eine Vielzahl von Herzperioden bezeichnend ist, und
Bestimmen des ersten Indexes auf der Grundlage der Vielzahl von AF-Intervallen;

und wobei
Bestimmen der Krankheit des Subjekts ferner auf dem ersten Index beruht.

3. Vorrichtung nach dem vorhergehenden Anspruch, wobei Bestimmen des ersten Indexes ferner umfasst:

Bestimmen eines Durchschnitts auf der Grundlage der Vielzahl von AF-Intervallen; und
Bestimmen des ersten Indexes auf der Grundlage des Durchschnitts.

4. Vorrichtung nach dem vorhergehenden Anspruch, wobei der Durchschnitt der quadratische Mittelwert der sukzessiven Differenz ist, wahlweise wobei Bestimmen des quadratischen Mittelwerts der sukzessiven Differenz auf der Grundlage der Atemfrequenzintervalle Normalisieren des quadratischen Mittelwerts der sukzessiven Differenz auf der Grundlage eines mittleren AF-Intervalls beinhaltet, das auf der Grundlage der Vielzahl von AF-Intervallen bestimmt wird.

5. Vorrichtung nach einem der vorhergehenden Ansprüche, wobei die Frequenzverteilung ein Histogramm umfasst, das für eine Vielzahl von Wahrscheinlichkeiten bezeichnend ist; wobei wahlweise der Entropiewert auf der Grundlage des Folgenden bestimmt wird:

$$S = -\sum_{i=1} p_i \cdot log_2(p_i),$$

wobei $p_i$ der Vielzahl von Wahrscheinlichkeiten entspricht; ferner wahlweise wobei das Histogramm eine Bingröße von 8 ms aufweist.

6. Vorrichtung nach einem der vorhergehenden Ansprüche, wobei Bestimmen des dritten Indexes umfasst:

Bestimmen einer Poincaré-Plot-Analyse (PPA) auf der Grundlage der Vielzahl von Herzschlagintervallen, wobei die Poincaré-Plot-Analyse bezeichnend für eine Zeitreihenschwankung ist, die auf der Grundlage einer jeweiligen Beziehung zwischen einem ersten Herzschlagintervall ($BBI_n$) und einem vorhergehenden zweiten Herzschlagintervall ($BBI_{n-1}$) der Vielzahl von Herzschlagintervallen bestimmt wird; und
Bestimmen des zweiten Indexes auf der Grundlage der Poincaré-Plot-Analyse.

7. Vorrichtung nach dem vorhergehenden Anspruch, wobei Bestimmen des dritten Indexes umfasst:

Bestimmen einer Standardabweichung SD1 einer kurzfristigen Variabilität des Herzschlagintervalls und einer Standardabweichung SD2 einer langfristigen Variabilität des Herzschlagintervalls; und
Bestimmen des zweiten Indexes auf der Grundlage eines Indexes SD1/SD2, der für ein Verhältnis der Standardabweichung SD1 zur Standardabweichung SD2 bezeichnend ist.

8. Vorrichtung nach einem der vorhergehenden Ansprüche, wobei die Pulswellendaten, die für eine Vielzahl von Herzperioden bezeichnend sind, sich auf eine Vielzahl direkt aufeinanderfolgender Herzperioden bezieht.

9. Vorrichtung nach einem der vorhergehenden Ansprüche, wobei der Teil der Pulswellendaten, der für eine Vielzahl von Herzperioden bezeichnend ist, eine Periode von mindestens 2 Minuten abdeckt; und
Bestimmen des zweiten und dritten Indexes und wahlweise des ersten Indexes im Wesentlichen auf allen Herzschlägen beruht, die in dem Teil der Pulswellendaten enthalten sind, der für eine Vielzahl von Herzperioden bezeichnend ist.

10. Vorrichtung nach dem vorhergehenden Anspruch, wobei der Teil der Pulswellendaten, die für eine Vielzahl von Herzperioden bezeichnend sind, eine Periode von mindestens 5 Minuten abdeckt.

11. Vorrichtung nach einem der vorhergehenden Ansprüche, wobei die Steuereinheit ferner zu Folgendem ausgelegt ist:
Bestimmen für jede Herzperiode der Vielzahl von Herzperioden, ob die jeweilige Herzperiode mit einer oder mehreren Störungen verbunden ist, auf der Grundlage der Pulswellendaten des Teils der Pulswellendaten, der für eine Vielzahl von Herzperioden bezeichnend ist, undÄndern der Pulswellendaten des Teils der Pulswellendaten, der für eine Vielzahl von Herzperioden bezeichnend ist, wenn die jeweilige Herzperiode mit einer oder mehreren Störungen verbunden ist, so dass die jeweilige Herzperiode nicht mehr mit einer oder mehreren Störungen verbunden ist.

**12.** Vorrichtung nach dem vorhergehenden Anspruch, wobei die eine oder mehreren Störungen einen verfrühten Herzschlag umfassen.


**Revendications**

**1.** Appareil pour déterminer une condition médicale d'un sujet humain, l'appareil comprenant :

une unité de commande ; et
un moyen pour fournir des données d'onde de pouls représentatives d'un battement de cœur du sujet humain ;
l'unité de commande étant configurée pour exécuter les étapes de :

réception des données d'onde de pouls ;
sélection d'une partie des données d'onde de pouls indicatives d'une pluralité de périodes cardiaques ;
détermination d'un deuxième et d'un troisième indice, chacun des deuxième et troisième indices étant basé sur les données d'onde de pouls de la partie des données d'onde de pouls indicatives d'une pluralité de périodes cardiaques, le troisième indice étant différent du deuxième indice ; et
détermination d'une condition médicale du sujet sur la base des deuxième et troisième indices ;
la détermination du deuxième indice comprenant :

la détermination d'un tachogramme indicatif d'une variabilité d'une pluralité d'intervalles de fréquence respiratoire sur la base des données d'onde de pouls de la partie des données d'onde de pouls indicatives d'une pluralité de périodes cardiaques,
la détermination d'une distribution de fréquence d'intervalles de fréquence respiratoire respectifs de la pluralité d'intervalles de fréquence respiratoire,
la détermination d'une valeur d'entropie basée sur la distribution de fréquence, et
la détermination du deuxième indice sur la base de la valeur d'entropie ; et

la détermination du troisième indice comprenant :

la détermination d'une pluralité d'intervalles battement à battement (BBI) sur la base des données d'onde de pouls de la partie des données d'onde de pouls indicatives d'une pluralité de périodes cardiaques, et
la détermination du troisième indice sur la base de la pluralité d'intervalles battement à battement.

**2.** Appareil selon la revendication précédente, comprenant en outre la détermination d'un premier indice, le premier indice étant différent des deuxième et troisième indices ;
la détermination du premier indice comprenant :

la détermination d'une pluralité d'intervalles RR sur la base des données d'onde de pouls de la partie des données d'onde de pouls indicatives d'une pluralité de périodes cardiaques, et
la détermination du premier indice sur la base de la pluralité d'intervalles RR ;

et
la détermination de la condition médicale du sujet étant en outre basée sur le premier indice.

**3.** Appareil selon la revendication précédente, la détermination du premier indice comprenant en outre :

la détermination d'une moyenne basée sur la pluralité d'intervalles RR ; et
la détermination du premier indice sur la base de la moyenne.

**4.** Appareil selon la revendication précédente, la moyenne étant la moyenne quadratique de la différence successive, optionnellement la détermination de la moyenne quadratique de la différence successive sur la base de la pluralité d'intervalles de fréquence respiratoire comprenant la normalisation de la moyenne quadratique de la différence successive sur la base d'un intervalle RR moyen déterminé sur la base de la pluralité d'intervalles RR.

5. Appareil selon l'une quelconque des revendications précédentes, la distribution de fréquence comprenant un histogramme indicatif d'une pluralité de probabilités ; éventuellement, la valeur d'entropie étant déterminée sur la base de :

$$S = -\sum_{i=1} p_i \cdot log_2(p_i),$$

$p_i$ correspondant à la pluralité de probabilités ; en outre, éventuellement, l'histogramme ayant une taille de case de 8 ms.

6. Appareil selon l'une quelconque des revendications précédentes, la détermination du troisième indice comprenant :

la détermination d'une analyse de tracé de Poincaré (PPA) sur la base de la pluralité d'intervalles battement à battement, l'analyse de tracé de Poincaré étant indicative d'une fluctuation de série temporelle déterminée sur la base d'une relation respective d'un premier intervalle battement à battement ($BBI_n$) et d'un deuxième intervalle battement à battement précédent ($BBI_{n-1}$) de la pluralité d'intervalles battement à battement ; et
la détermination du deuxième indice sur la base de l'analyse du tracé de Poincaré.

7. Appareil selon la revendication précédente, la détermination du troisième indice comprenant :

la détermination d'un écart type SD1 d'une variabilité d'intervalle battement à battement à court terme et d'un écart type SD2 d'une variabilité d'intervalle battement à battement à long terme ; et
la détermination du deuxième indice sur la base d'un indice SD1/SD2 indiquant un rapport de l'écart type SD1 sur l'écart type SD2.

8. Appareil selon l'une quelconque des revendications précédentes, les données d'onde de pouls indicatives d'une pluralité de périodes cardiaques concernant une pluralité de périodes cardiaques en succession directe les unes par rapport aux autres.

9. Appareil selon l'une quelconque des revendications précédentes, la partie des données d'onde de pouls indicatives d'une pluralité de périodes cardiaques couvrant une période d'au moins 2 minutes ; et
la détermination des deuxième et troisième indices, et éventuellement du premier indice, étant basée sur sensiblement tous les battements cardiaques compris dans la partie des données d'onde de pouls indicatives d'une pluralité de périodes cardiaques.

10. Appareil selon la revendication précédente, la partie des données d'onde de pouls indicatives d'une pluralité de périodes cardiaques couvrant une période d'au moins 5 minutes.

11. Appareil selon l'une quelconque des revendications précédentes, l'unité de commande étant en outre configurée pour :

déterminer, sur la base des données d'onde de pouls de la partie des données d'onde de pouls indicatives d'une pluralité de périodes cardiaques, pour chaque période cardiaque de la pluralité de périodes cardiaques, si la période cardiaque respective est associée avec une ou plusieurs perturbations, et
modifier les données d'onde de pouls de la partie des données d'onde de pouls indicatives d'une pluralité de périodes cardiaques si la période cardiaque respective est associée avec une ou plusieurs perturbations de sorte que la période cardiaque respective ne soit plus associée avec la ou les perturbations.

12. Appareil selon la revendication précédente, la ou les perturbations comprenant un battement prématuré.

# FIG.1

400 — ( START )

Record video signal indicative of
acral blood flow using video sensor and light
source of mobile device — 402

Choose region of interest
of at least 50x50 pixels — 404

Eliminate 1st Bit of green value of pixel
and calculate sum of values for each frame
to obtain associated sample — 406

Obtain time stamp for region of interest
based on respective video frame — 408

Obtain pulse wave signal based on sample — 410

Re-sample pulse wave signal based on samples
and time stamps using a cubic spline interpolation
in order to obtain re-sampled pulse wave signal — 412

Filter re-sampled pulse wave signal to
eliminate noise and to compensate for drift — 414

Obtain original pulse wave signal
for processing — 416

418 — ( END )

FIG.2A

FIG.2B

EP 3 117 767 B1

FIG.3A

FIG.3B

# FIG.3C

300 — START

Recording
pulse wave data — 302

Determining suitable
heart rate periods — 304

Decomposition of each heart period
into systolic and diastolic components — 306

Approximation of the systolic component
with at least two Gaussian functions by means of
fitting against the original pulse wave — 308

Determining the time difference
between the fitted Gaussian functions — 310

Determining the SI from the height of
the subject and the time difference — 312

Compensating the determined SI value
based on the age of the subject by means of
known linear dependencies — 314

Estimation of the effective blood pressure
as a function of the compensated SI by means of
gender-specific regression models — 316

318 — END

RESPIRATORY RATE
& VARIATION

207

RESPIRATION

201

MEASURED
PULSE WAVE

BLOOD PRESSURE
& VARIATION

HEART RATE
& VARIATION

208

206

FIG.3D

100 —— START

Determine suitable heart rate periods —— 102

Determine beat-to beat intervals BBI based on heart periods —— 104

Determine 1$^{st}$ index indicative of heart rate variability —— 106

Determine 2$^{nd}$ index indicative of heart rate variability —— 108

Determine medical condition based on 1$^{st}$ and 2$^{nd}$ indexs —— 110

112 —— END

FIG.3E

EP 3 117 767 B1

(A) normalized RMSSD

Normalized units

2.0

1.5

1.0

0.5

0.0

SR          AF

95% Confidence intervall

**FIG.4A**

(B) Shannon Entropy

Units (bit)

5.5

5.0

4.5

4.0

3.5

SR          AF

95% Confidence intervall

**FIG.4B**

(C) SD1/SD2

1.0

0.8

0.6

0.4

0.2

**SR**          AF

95% Confidence intervall

**FIG.4C**

FIG.5A

FIG.5B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- GB 25059890 A

- WO 2009152521 A

### Non-patent literature cited in the description

- **GLADSTONE DJ ; SPRING M ; DORIAN P ; PAN-ZOV V ; THORPE KE ; HALL J et al.** Atrial fibrillation in patients with cryptogenic stroke. *The New England journal of medicine,* 2014, vol. 370, 2467-2477
- **SANNA T ; DIENER HC ; PASSMAN RS ; DI LAZ-ZARO V ; BERNSTEIN RA ; MORILLO CA et al.** Cryptogenic stroke and underlying atrial fibrillation. *N Engl J Med,* 2014, vol. 370, 2478-2486
- **HEALEY JS ; CONNOLLY SJ ; GOLD MR ; ISRAEL CW ; VAN GELDER IC ; CAPUCCI A et al.** Subclinical atrial fibrillation and the risk of stroke. *N Engl J Med,* 2012, vol. 366, 120-129
- **ZIEGLER PD ; GLOTZER TV ; DAOUD EG ; WYSE DG ; SINGER DE ; EZEKOWITZ MD et al.** Incidence of newly detected atrial arrhythmias via implantable devices in patients with a history of thromboembolic events. *Stroke,* vol. 41, 256-260

- **A. SEECK ; W. RADEMACHER ; C. FISCHER ; J. HAUEISEN ; R. SURBER ; A. VOSS.** *Prediction of atrial fibrillation recurrence after cardioversion-Interaction analysis of cardiac autonomic regulation*
- **W. POPPE et al.** *Eignen sich die Hüllungskurven von Arterienpulswellen für eine Fernbeurteilung psychotischer Krankheitsverläufe?*
- **GREEN GEOFFREY C. et al.** Continuous Multiorgan Variability Analysis To Track Severity Of Organ Failure In Critically III Patients. *Journal Of Critical Care,* 31 October 2013, vol. 28 (5
- Automatic Real Time Detection Of Atrial Fibrillation. **DASH S. et al.** Annals Of Biomedical Engineering. Kluwer Academic Publishers-Plenum Publishers, 17 June 2009, vol. 37
- **BRISINDA D. et al.** Discriminant Analysis Of Heart Rate Variability After Electrical Cardioversion Predicts Atrial Fibrillation Recurrence. *International Journal Of Clinical Cardiology,* 25 November 2014